# EUROPEAN PATENT APPLICATION

(11) **EP 4 317 434 A1**
(43) Date of publication of application: **07.02.2024**
(21) Application number: 22779001.1
(22) Date of filing: 30.03.2022
(51) Int. Cl.: C12N 15/113, C12N 7/00, A61K 48/00

(54) **RNA DELIVERY SYSTEM FOR TREATMENT OF HUNTINGTON'S DISEASE**

(30) Priority: 30.03.2021 CN 202110341397
(71) Applicant: Nanjing University, Nanjing, Jiangsu 210023 (CN)
(72) Inventor: ZHANG, Chenyu, Nanjing, Jiangsu 210023 (CN); CHEN, Xi, Nanjing, Jiangsu 210023 (CN); FU, Zheng, Nanjing, Jiangsu 210023 (CN); LI, Jing, Nanjing, Jiangsu 210023 (CN); ZHANG, Xiang, Nanjing, Jiangsu 210023 (CN); ZHOU, Xinyan, Nanjing, Jiangsu 210023 (CN); ZHANG, Li, Nanjing, Jiangsu 210023 (CN); YU, Mengchao, Nanjing, Jiangsu 210023 (CN); GUO, Hongyuan, Nanjing, Jiangsu 210023 (CN)
(74) Representative: Cabinet Laurent & Charras
(86) International application number: PCT/CN2022/083992
(87) International publication number: WO 2022/206819

(57) **Abstract**

An RNA delivery system for the treatment of Huntington's disease. The system comprises a viral vector, the viral vector carries RNA fragments capable of treating Huntington's disease, the viral vector is capable of enrichment in organ tissues of a host and endogenously and spontaneously forming a complex containing the RNA fragments capable of treating Huntington's disease in the organ tissues of the host, and the complex can deliver the RNA fragments into a target tissue to treat Huntington's disease. The safety and reliability of the RNA delivery system for the treatment of Huntington's disease have been fully verified. The system has good medicinal properties, strong versatility, and has economic benefits and application prospects.

## Description

### FIELD

The application relates to the technical field of biomedicine, and specifically relates to an RNA delivery system for the treatment of Huntington's disease.

### BACKGROUND

Huntington's disease (HD), also known as chorea or Huntington's chorea, is an autosomal dominant genetic neurodegenerative disease. The primary cause is a mutation in the Huntington gene located on chromosome 4 of the patient, resulting in the production of a mutated protein that gradually aggregates within cells, forming large molecular clusters that accumulate in the brain, affecting the function of nerve cells. Typically, patients develop symptoms in middle age, characterized by chorea-like movements, and as the disease progresses, they gradually lose the ability to speak, move, think, and swallow, with the condition lasting approximately 10 to 20 years and ultimately leading to the patient's death.

RNA interference (RNAi) therapy has been considered a promising strategy to treat human diseases since its invention. However, it has faced numerous issues during clinical practice, and the progress of the therapy's development has fallen short of expectations.

It is generally believed that RNA cannot remain stable outside the cell for a long time, because RNA will be degraded into fragments due to the abundance of RNase in the extracellular environment. It is therefore crucial to find a method that can stabilize RNA outside the cell and enable it to enter targeted tissues, thereby enhancing the effect of RNAi therapy.

There are currently several patents concerning siRNA, mainly focusing on the following aspects: 1. Designing medically effective siRNA. 2. Chemically modifying siRNA in order to enhance its stability in vivo and increase the yield. 3. Refining the design of various artificial carriers, including lipid nanoparticles, cationic polymers and viruses, to improve the efficiency of siRNA delivery in vivo. Among them, there are a number of patents regarding the third aspect. The primary cause for this is that researchers have realized the absence of suitable siRNA delivery systems for safely, accurately and efficiently deliver siRNA to target tissues. This challenge has become the fundamental limitation of RNAi therapy.

Biological virus is a kind of noncellular organism with small body and simple structure, which contains only one kind of nucleic acid (DNA or RNA) and must be parasitic in living cells and proliferated by replication. Viral vectors can bring genetic materials into cells. The principle is that viruses have the molecular mechanism of transferring their genomes into other cells for infection, which can occur in intact living bodies (in vivo) or cell cultures (in vitro), and are mainly used in basic research, gene therapy or vaccines. However, at present, there is little research on the delivery of RNA, especially siRNA, by using virus as a carrier and using special self-assembly mechanism.

Chinese patent CN108624590A discloses an siRNA capable of inhibiting the expression of DDR2 gene. Chinese patent CN108624591A discloses an siRNA capable of silencing ARPC4 gene, and the siRNA is modified with α-phosphorus-selenium. Chinese patent CN108546702A discloses an siRNA targeting a long non-coding RNA, DDX11-AS1. Chinese patent CN106177990A discloses an siRNA precursor that can be used for the treatment of various tumors. These patents devise specific siRNAs and target certain diseases caused by genetic mutations.

Chinese patent CN108250267A discloses a polypeptide and a polypeptide-siRNA induced co-assembly, where the polypeptide acts as a carrier of siRNA. Chinese patent CN108117585A discloses a polypeptide to target and to introduce siRNA for promoting apoptosis of breast cancer cells, also utilizing the polypeptide as a carrier of siRNA. Chinese patent CN108096583A discloses a nanoparticle carrier, which can be loaded with siRNA that has curative effect on breast cancer while containing chemotherapeutic medicaments. These patents are all inventions on siRNA carriers, whose technical solutions share the common feature of preassembling the carrier and siRNA *in vitro* before introducing them to the host. In fact, this is characteristic for most of the currently designed delivery techniques. However, these delivery systems pose a common issue that such artificially synthesized exogenous delivery system is prone to be cleared by the host's circulatory system, cause immunogenic reactions, and even potentially be toxic to certain cell types and tissues.

The research team of the present invention found that endogenous cells can selectively encapsulate miRNAs into exosomes. Exosomes can deliver miRNA to receptor cells, and the secreted miRNA can effectively block the expression of target genes at a relatively low concentration. Exosomes are biocompatible with the host immune system and possess the inherent ability to protect and transport miRNA across biological barriers in vivo, thereby having the potential to overcome problems associated with siRNA delivery. For example, Chinese patent CN110699382A discloses a method for preparing exosomes delivering siRNA, which involves techniques of isolating exosomes from plasma and encapsulating siRNA into exosomes by electroporation.

However, such techniques for isolating or preparing exosomes in vitro often necessitate obtaining a large amount of exosomes through cell culture, together with the step of siRNA encapsulation, which makes the clinical cost of large-scale application of the product too high for patients to afford. More importantly, the intricate production/purification process of exosomes makes it almost impossible to comply with GMP standards.

So far, medicinal products containing exosomes as active ingredients have not received approval from CFDA. The main challenge lies in ensuring the consistency of exosome products, which results in these products failing to obtain medicament production licenses. If this issue can be resolved, it would significantly advance RNAi therapy for the treatment of Huntington's disease.

Therefore, the development of a safe, precise and efficient siRNA delivery system is a crucial part to improve the efficacy of RNAi therapy and advance it further.

### SUMMARY

In view of this, the embodiments of the present application provides an RNA delivery system for the treatment of Huntington's disease and a use thereof to solve the technical deficiencies existing in the existing technology.

An inventive point of the present application is to provide an RNA delivery system for the treatment of Huntington's disease, the system comprises a viral vector, the viral vector carries RNA fragments capable of treating Huntington's disease, the viral vector is capable of enrichment in organ tissues of a host and endogenously and spontaneously forming a complex containing the RNA fragments capable of treating Huntington's disease in the organ tissues of the host, and the complex can deliver the RNA fragments into a target tissue to treat Huntington's disease. After the RNA fragments are delivered to brain, a target tissue, they can inhibit the expression of the matching genes, and then inhibit the development of diseases in the target tissue.

Further, the virus vector is an adenovirus-associated virus.

Further, the adenovirus-associated virus is adenovirus-associated virus type 5, adenovirus-associated virus type 8 or adenovirus-associated virus type 9.

Further, the RNA fragment contains one, two or more specific RNA sequences with medical significance, and the RNA sequence is siRNA, shRNA or miRNA with medical significance.

Further, the viral vector comprises a promoter and a targeting tag, the targeting tag is capable of forming a targeting structure for the complex in the organ tissues of the host, the targeting structure is located on the surface of the complex, the complex can find and bind to target tissues through the targeting structure, to deliver the RNA fragments into the target tissues.

Further, the viral vector comprises any one of the following circuits or the combination of several circuits: promoter-RNA fragment, promoter-targeting tag, promoter-RNA fragment-targeting tag; each viral vector comprises at least one RNA fragment and one targeting tag, and the RNA fragment and targeting tag are located in the same circuit or different circuits.

Further, the viral vector further comprises a flanking sequence, a compensation sequence and a loop sequence that facilitate the folding into a correct structure and the expression of the circuit, the flanking sequences comprises 5' flanking sequences and 3' flanking sequences;

The viral vector comprises any one of the following circuits or the combination of several circuits: 5'-promoter-5' flanking sequence-RNA fragment-loop sequence-compensation sequence-3' flanking sequence, 5'-promoter-targeting tag, 5'-promoter-targeting tag-5' flanking sequence-RNA fragment-loop sequence-compensation sequence-3' flanking sequence.

Further, the 5' flanking sequence has a sequence that is identical to or more than 80% homologous with ggatcctggaggcttgctgaaggctgtatgctgaattc;

The loop sequence has a sequence that is identical to or more than 80% homologous with gttttggccactgactgac;

The 3' flanking sequence has a sequence that is identical to or more than 80% homologous with accggtcaggacacaaggcctgttactagcactcacatggaacaaatggcccagatctggccgcactcgag;

The compensation sequence has a reverse complementary sequence to the RNA fragment in which any 1 to 5 bases have been deleted. The purpose of deleting 1 to 5 bases of RNA reverse complementary sequence is to make the sequence not express.

Different from shRNA, in the precursor of siRNA and miRNA, the bases at positions 9 and 10 of the reverse complementary chain to the functional chain (for example, the reverse complementary chain to siRNA) are deleted, which leads to the formation of a bulge structure of the functional chain of siRNA and miRNA, which is conducive to more effective silencing of gene expression. Therefore, it is an accepted conclusion to improve the silencing efficiency by deleting the bases at positions 9 and 10 of the reverse complementary sequences.

Preferably, the compensation sequence is the reverse complementary sequence of the RNA fragment in which any 1 to 3 bases have been deleted.

More preferably, the compensation sequence is the reverse complementary sequence of the RNA fragment in which any 1 to 3 consecutively arranged bases have been deleted.

Most preferably, the compensation sequence is the reverse complementary sequence of the RNA fragment in which bases at positions 9 and/or 10 have been deleted.

Further, in the case where there are at least two circuits in the virus vector, adjacent circuits are connected by a sequence consisting of sequences 1-3 (sequence 1- sequence 2-sequence 3);

Wherein, sequence 1 is CAGATC, sequence 2 is a sequence consisting of 5-80 bases, and sequence 3 is TGGATC.

Further, in the case where there are at least two circuits in the virus vector, adjacent circuits are connected by a sequence that is identical to or more than 80% homologous with sequence 4;

Wherein, the sequence 4 is CAGATCTGGCCGCACTCGAGGTAGTGAGTCGACCAGTGGATC.

Further, the organ tissue is liver, and the complex is exosome.

Further, the targeting tag is selected from targeting peptides or targeting proteins with targeting functions.

Further, the targeting peptides include RVG targeting peptide, GE11 targeting peptide, PTP targeting peptide, TCP-1 targeting peptide, MSP targeting peptide;

The targeting proteins include RVG-LAMP2B fusion protein, GE11-LAMP2B fusion protein, PTP-LAMP2B fusion protein, TCP-1-LAMP2B fusion protein, MSP-LAMP2B fusion protein.

The targeting tag is preferably an RVG targeting peptide or an RVG-LAMP2B fusion protein which can accurately target brain tissue.

Further, the length of the RNA sequence is 15-25 nucleotides. For example, the length of the RNA sequence can be 16, 17, 18, 19, 20, 21, 22, 23, 24 and 25 nucleotides. Preferably, the length of the RNA sequence is 18-22 nucleotides.

Further, the RNA capable of treating Huntington's disease is selected from siRNA of mHTT gene, or a sequence that is identical to or more than 80% homologous with the above sequence, or a nucleic acid molecule encoding the above RNA. It should be noted that the RNA sequence in the "nucleic acid molecule encoding the above RNA sequence" here also includes RNA sequences that are identical to or more than 80% homologous with each RNA.

The siRNA of the mHTT gene includes UAUGUUUUCACAUAUUGUCAG, AUUUAGUAGCCAACUAUAGAA, AUGUUUUUCAAUAAAUGUGCC, UAUGAAUAGCAUUCUUAUCUG, UAUUUGUUCCUCUUAAUACAA, other sequences that inhibit mHTT gene expression, and sequences that are identical to or more than 80% homologous with the above sequences.

It should be noted that the above-mentioned "sequences that are more than 80% homologous with" may refer to a homology of 85%, 88%, 90%, 95%, 98%, etc.

Optionally, the RNA fragment includes the RNA sequence and a modified RNA sequence obtained by ribose modification to the RNA sequence. That is to say, the RNA fragment may consist of at least one RNA sequence, at least one modified RNA sequence, or the RNA sequence and the modified RNA sequence.

In the present invention, the isolated nucleic acid also includes variants and derivatives thereof. Those skilled in the art can use common methods to modify the nucleic acid. Such modification includes (but not limited to) methylation modification, hydrocarbon modification, glycosylation modification (such as 2-methoxy-glycosyl modification, hydroxyl-glycosyl modification, saccharide ring modification), nucleic acid modification, peptide fragment modification, lipid modification, halogen modification, and nucleic acid modification (such as "TT" modification). In one embodiment of the present invention, the modification is an internucleotide linkage, for example selected from the group consisting of phosphorothioate, 2'-O methoxyethyl (MOE), 2'-fluoro, alkyl phosphonate, phosphorodithioate, alkyl phosphonothioate, phosphoramidate, carbamate, carbonate, phosphotriester, acetamidate, carboxymethyl ester, and combinations thereof. In one embodiment of the present invention, the modification is a modification of nucleotides, for example, selected from the group consisting of peptide nucleic acid (PNA), locked nucleic acid (LNA), arabinose nucleic acid (FANA), analogs and derivatives thereof, and combinations thereof. Preferably, the modification is 2' fluoropyrimidine modification. 2' Fluoropyrimidine modification is to replace the 2'-OH of the pyrimidine nucleotide of RNA with 2'-F. 2'-F modification can make RNA difficult to be recognized by RNase in vivo, thereby increasing the stability of RNA fragment during transportation in vivo.

Further, the delivery system is a delivery system for use in a mammal including human.

Another inventive point of the present application is to provide use of the RNA delivery system for the treatment of Huntington's disease in the manufacture of a medicament for treating a disease.

The medicament is a medicament for the treatment of Huntington's disease and related diseases, here the related diseases refer to related diseases or complications, sequelae, etc. occurring during the formation or development of the above Huntington's disease, or other diseases with certain correlation with Huntington's disease.

Further, the medicament comprises the above-mentioned viral vector, specifically, the viral vector here means a viral vector carrying RNA fragments or RNA fragments and targeting tags, and can enter the host body and be enriched in the liver, and self-assemble to form a complex exosome. The complex can deliver the RNA fragments into at target tissue, so that the RNA fragments can be expressed in the target tissue, thereby inhibiting the expression of the matching genes and achieving the purpose of treating diseases.

The dosage form of the medicament may be tablets, capsules, powders, granules, pills, suppositories, ointments, solutions, suspensions, lotions, gels, pastes, etc.

Further, the medicament is administered by oral administration, inhalation, subcutaneous injection, intramuscular injection, or intravenous injection.

### The technical effects of the present application include:

The RNA delivery system for the treatment of Huntington's disease provided in this application takes the virus as a carrier. As a mature injection material, the virus vector has been fully verified for its safety and reliability, and it exhibits excellent medicament-making property. The RNA sequences that ultimately effect are delivered enclosed within endogenous exosomes, leading to the absence of any immune reactions, eliminating the need to validate the safety of these exosomes. The delivery system can deliver all kinds of small molecular RNA with strong versatility. Moreover, the preparation of virus vectors is much cheaper and more economical than the preparation of exosomes, proteins, polypeptides and other substances. After self-assembly in vivo, the RNA delivery system for the treatment of Huntington's disease provided in the present application can tightly binds with AGO₂ and enriched as a complex (exosome), which not only can prevent its premature degradation and maintain its stability in circulation, but also is beneficial to the absorption of recipient cells, cytoplasmic release and lysosomal escape, and only a low dose is required.

The RNA delivery system for the treatment of Huntington's disease provided in the present application is used in the manufacture of a medicament for treating a disease, that is, it provides a medicament delivery platform, which can greatly improve the therapeutic effect for Huntington's disease, and can also form the research and development basis of more RNA medicaments through this platform. This significantly advances the development and utilization of RNA medicaments.

### BRIEF DESCRIPTION OF DRAWINGS

Fig. 1 shows the comparison of the treatment of Huntington's disease in mice provided by an example of the present application;
Fig. 2 shows the comparison of the descending latency of mice provided by an example of the present application.
Fig. 3 shows the effect of in vivo enrichment of a lentiviral vector loaded with siRNA provided by an example of the present application.
Fig. 4 shows the effect of in vivo self-assembly of a lentiviral vector loaded with siRNA provided by an example of the present application.
Fig. 5 shows the electrophoresis result showing that the lentiviral vector loaded with siRNA provided by an example of the present application has therapeutic effect on Huntington's disease.
Fig. 6 shows the effect of in vivo enrichment of adenovirus-associated virus type 1, type 4 and type 7 vectors loaded with siRNA provided by an example of the present application.
Fig. 7 shows the electrophoresis result showing that adenovirus-associated virus type 1, type 2 and type 7 vectors loaded with siRNA provided by an example of the present application have therapeutic effects on Huntington's disease.
Fig. 8 shows the data of the therapeutic effect of adenovirus-associated virus type 1, type 2 and type 7 vectors loaded with siRNA provided by an example of the present application on Huntington's disease, in which the comparison was made with HTT mRNA level.
Fig. 9 shows the effect of in vivo enrichment of adenovirus-associated virus type 8 vector loaded with six separate RNA sequences provided by an example of the present application.
Fig. 10 shows the effect of in vivo enrichment of adenovirus-associated virus type 9 vector loaded with six separate RNA sequences provided by an example of the present application.
Fig. 11 shows the electrophoresis result showing that adenovirus-associated virus type 9 vector loaded with six separate RNA sequences provided by an example of the present application has therapeutic effect on Huntington's disease.
Fig. 12 shows the data of the therapeutic effect of adenovirus-associated virus type 8 vector loaded with RNA fragments of any two RNA sequences provided by an example of the present application on Huntington's disease, in which the comparison was made with HTT mRNA level.
Fig. 13 shows the electrophoresis result showing that adenovirus-associated virus type 9 vector loaded with RNA fragments of any two RNA sequences provided by an example of the present application has therapeutic effect on Huntington's disease.
Fig. 14 shows the data of the therapeutic effect of adenovirus-associated virus type 8 vector loaded with RNA fragments of any three RNA sequences provided by an example of the present application on Huntington's disease, in which the comparison was made with HTT mRNA level.
Fig. 15 shows the electrophoresis result showing that adenovirus-associated virus type 9 vector loaded with RNA fragments of any three RNA sequences provided by an example of the present application has therapeutic effect on Huntington's disease.
Fig. 16 shows the in vivo enrichment data in the case where the sequence of adenovirus-associated virus type 9 provided by an example of the present application contains siRNA and RVG.
Fig. 17 shows the therapeutic effect on Huntington's disease by the expression data of HTT mRNA in the case where the sequence of adenovirus-associated virus type 9 provided by an example of the present application contains siRNA and RVG.
Fig. 18 shows the data comparison on the therapeutic effect on Huntington's disease of RVG-LAMP2B fusion protein and other fusion proteins in adenovirus vector provided by an example of the present application, and the data is reflected by the relative level of HTT mRNA.
Fig. 19 shows the in vivo enrichment data in the case where the adenovirus vector system provided by an example of the present application comprises three RNA sequences with more than 80% homology with the siRNA sequence of the HTT gene.
Fig. 20 shows the data comparison on the therapeutic effect on Huntington's disease of the adenovirus vector system provided by an example of the present application in the case where the adenovirus vector system comprises three RNA sequences with more than 80% homology with the siRNA sequence of the HTT gene, and the data is reflected by the relative level of HTT mRNA.

### DETAILED DESCRIPTION

Specific embodiments of the present application will be described below in conjunction with the accompanying drawings.

The detection of siRNA, protein and mRNA levels involved in the present invention is all performed by injecting an RNA delivery system into mice to establish an in vitro mouse stem cell model. qRT-PCR is used to detect the expression levels of mRNA and siRNA in cells and tissues. The absolute quantification of siRNA is determined by drawing a standard curve with a standard product. The expression level of each siRNA or mRNA relative to the internal reference can be represented by 2- Δ CT, where Δ CT=Csample-Cinternal reference. The internal reference gene used is U6 snRNA (in tissue) or miR-16 (in serum, exosomes) molecules when amplifying siRNA, and is GAPDH or 18s RNA when amplifying mRNA. Western blotting is used to detect the expression level of proteins in cells and tissues, and ImageJ software is used for protein quantitative analysis.

In the figures provided in the present invention, * means P < 0.05, * * means p < 0.01, and * * * means P< 0.005.

In the present invention, unless otherwise specified, the scientific and technical terms used herein have the meanings commonly understood by those skilled in the art. Moreover, the reagents, materials and procedures used herein are all reagents, materials and procedures widely used in the corresponding fields.

### Example 1

This example provides an RNA delivery system for the treatment of Huntington's disease. The system comprises a viral vector, the viral vector carries RNA fragments capable of treating Huntington's disease, the viral vector is capable of enrichment in organ tissues of a host and endogenously and spontaneously forming a complex containing the RNA fragments capable of treating Huntington's disease in the organ tissues of the host, and the complex can deliver the RNA fragments into a target tissue to treat Huntington's disease.

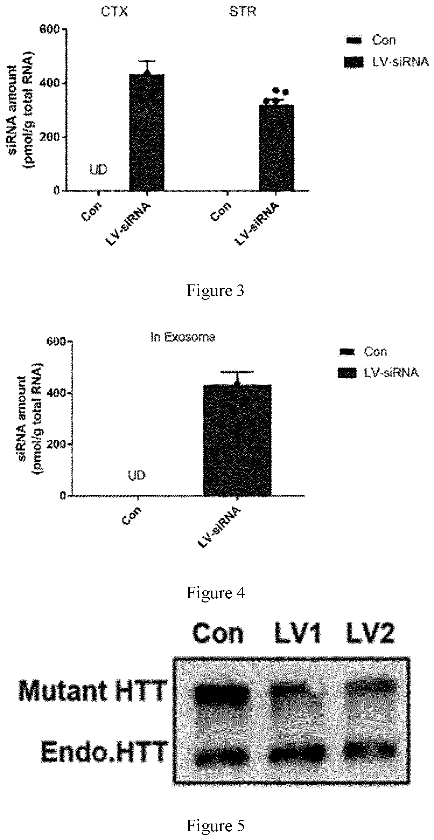

In addition to adenovirus, other virus vectors also have in vivo enrichment, self-assembly and therapeutic effects on Huntington's disease, such as lentivirus. The data of lentivirus are shown in Figures 3-5, where CTX stands for cortex and STR stands for Striatum.

The virus vector is preferably adenovirus-associated virus, more preferably adenovirus-associated virus type 5, adenovirus-associated virus type 8 or adenovirus-associated virus type 9.

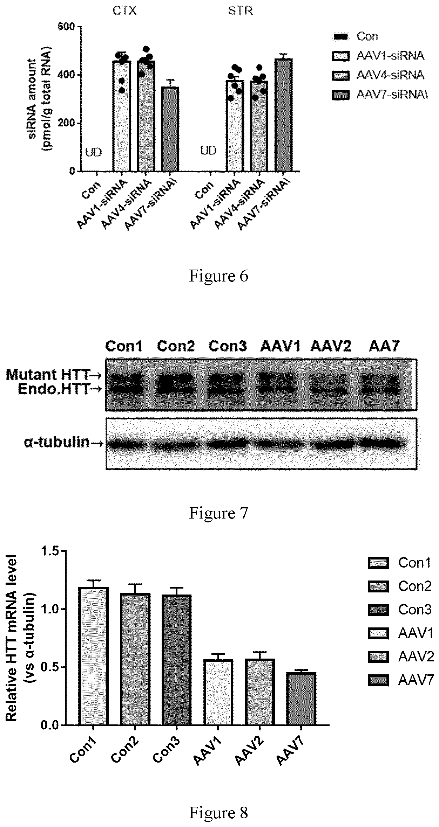

In addition to adenovirus-associated viruses types 5, 8 and 9, other virus vectors also have in vivo enrichment, self-assembly and therapeutic effects on Huntington's disease, such as adenovirus types 1, 2, 4 and 7, and the data of them are shown in Figures 6-8.

In this example, the viral vector further comprises a promoter and a targeting tag. The virus vectors comprise any one of the following circuits or the combination of several circuits: promoter -RNA sequence, promoter-targeting tag, promoter -RNA sequence-targeting tag, and each virus vector comprises at least one RNA fragment and one targeting tag, and the RNA fragment and the targeting tag are located in the same circuit or different circuits. In other words, the virus vector can comprise only the promoter -RNA sequence-targeting tag, or it can comprise the combination of promoter -RNA sequence and promoter-targeting tag, or the combination of promoter-targeting tag and promoter -RNA sequence-targeting tag.

Further, the viral vector can further comprises a flanking sequence, a compensation sequence and a loop sequence that facilitate the folding into a correct structure and the expression of the circuit, the flanking sequences comprises 5' flanking sequences and 3' flanking sequences; the viral vector comprises any one of the following circuits or the combination of several circuits: 5'-promoter-5' flanking sequence-RNA fragment-loop sequence-compensation sequence-3' flanking sequence, 5'-promoter-targeting tag, 5'-promoter-targeting tag-5' flanking sequence-RNA fragment-loop sequence-compensation sequence-3' flanking sequence.

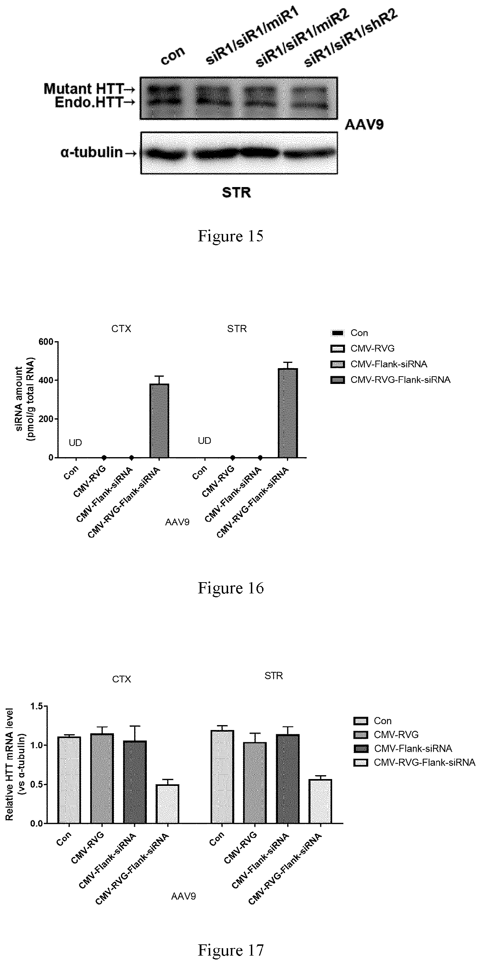

In the case where the virus vector comprises 5'- promoter-targeting tag -5' flanking sequence -RNA fragment -loop sequence-compensation sequence -3' flanking sequence, the in vivo enrichment and treatment effect on Huntington's disease of its RNA delivery system are shown in Figures 16-17, the targeting tag is RVG, and the RNA fragment is siRNA with therapeutic effect.

Wherein, the 5' flanking sequence preferably has a sequence that is identical to or more than 80% homologous with ggatcctggaggcttgctgaaggctgtatgctgaattc, including sequences with 85%, 90%, 92%, 95%, 98%, or 99% homology with ggatcctggaggcttgctgaaggctgtatgctgaattc.

The loop sequence preferably has a sequence that is identical to or more than 80% homologous with gttttggccactgactgac, including sequences with 85%, 90%, 92%, 95%, 98%, 99% homology with gttttggccactgactgac.

The 3' flanking sequence preferably has a sequence that is identical to or more than 80% homologous with accggtcaggacacaaggcctgttactagcactcacatggaacaaatggcccagatctggccgcactcgag, including sequences with 85%, 90%, 92%, 95%, 98%, 99% homology with accggtcaggacacaaggcctgttactagcactcacatggaacaaatggcccagatctggccgcactcgag.

The compensation sequence is the reverse complementary sequence of the RNA fragment in which any 1 to 5 bases have been deleted. In the case where the RNA fragment contains only one RNA sequence, the compensation sequence may be the reverse complementary sequence of the RNA sequence in which any 1 to 5 bases have been deleted.

Preferably, the compensation sequence is the reverse complementary sequence of the RNA fragment in which any 1 to 3 bases have been deleted. In the case where the RNA fragment contains only one RNA sequence, the compensation sequence may be the reverse complementary sequence of the RNA sequence in which any 1 to 3 bases have been deleted.

More preferably, the compensation sequence is the reverse complementary sequence of the RNA fragment in which any 1 to 3 consecutively arranged bases have been deleted. In the case where the RNA fragment contains only one RNA sequence, the compensation sequence may be the reverse complementary sequence of the RNA sequence in which any 1 to 3 consecutively arranged bases have been deleted.

Most preferably, the compensation sequence is the reverse complementary sequence of the RNA fragment in which bases at positions 9 and/or 10 have been deleted. In the case where the RNA fragment contains only one RNA sequence, the compensation sequence may be a reverse complementary sequence of the RNA sequence in which bases at positions 9 and/or 10 have been deleted. Deleting the bases at positions 9 and 10 has the best effect.

It should be noted that the above-mentioned flanking sequence, compensation sequence, and loop sequence are not randomly selected, but are determined based on a large number of theoretical studies and experiments. With the cooperation of the above-mentioned certain flanking sequence, compensation sequence, and loop sequence, the expression rate of RNA fragments can be maximized.

In the case where the virus vector carries two or more circuits, adjacent circuits can be connected by sequence 1- sequence 2- sequence 3; wherein, sequence 1 is preferably CAGATC, and sequence 2 can be a sequence consisting of 5-80 bases, such as 10, 15, 20, 25, 30, 35, 40, 45, 50, 55, 60, 65, 70 and 75 bases, preferably a sequence consisting of 10-50 bases, more preferably a sequence consisting of 20-40 bases, and sequence 3 is preferably TGGATC.

Sequence 2 is shown in Table 1 below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAGTG |
| 60 bases | TGGCCGCACTCGAGGTAGTGAGTCGACCAGTGGCCGCACTCGAGGTAGTGAGTCGAC CAG |
| 70 bases | TGGCCGCACTCGAGGTAGTGAGTCGACCAGTGGCCGCACTCGAGGTAGTGAGTCGAC CAGTGGCCGCACT |
| 80 bases | TGGCCGCACTCGAGGTAGTGAGTCGACCAGTGGCCGCACTCGAGGTAGTGAGTCGAC CAGTGGCCGCACTCGAGGTAGTG |

More preferably, in the case where the virus vector carries two or more circuits, adjacent circuits are connected by sequence 4 or a sequence that is identical to or more than 80% homologous with sequence 4; wherein, sequence 4 is CAGATCTGGCCGCACTCGAGGTAGTGAGTCGACCAGTGGATC.

The sequence is shown in Table 2 below.

| | |
|---|---|
| Sequence 4 | CAGATCTGGCCGCACTCGAGGTAGTGAGTCGACCAGTGGATC |
| Sequence 4-1 | CAGATCTGGCCGCACTCGTAGAGGTGAGTCGACCAGTGGATC |
| Sequence 4-2 | CAGATCTGGCACCCGTCGAGGTAGTGAGTCGACCAGTGGATC |
| Sequence 4-3 | CAGATCTGGCCGCACAGGTCGTAGTGAGTCGACCAGTGGATC |

The RNA fragment mentioned above contains one, two or more specific RNA sequences with medical significance, the RNA sequences can be expressed in the target recipient, but the compensation sequence cannot be expressed in the target recipient. RNA sequence can be siRNA sequence, shRNA sequence or miRNA sequence, preferably siRNA sequence.

The length of an RNA sequence is 15-25 nucleotides (nt), preferably 18-22 nt, such as 18 nt, 19 nt, 20 nt, 21 nt, or 22 nt. The range of the sequence length is not chosen arbitrarily but was determined after repeated trials. A large number of experiments have proved that in the case where the length of the RNA sequence is less than 18 nt, especially less than 15 nt, the RNA sequence is mostly invalid and will not play a role. In the case where the length of the RNA sequence is greater than 22 nt, especially greater than 25 nt, not only does the cost of the circuit increase greatly, but the effect is no better than that of an RNA sequence with a length of 18-22 nt, and the economic benefits are poor. Therefore, in the case where the length of the RNA sequence is 15-25 nt, especially 18-22 nt, the cost and function can be balanced with the best effect.

The RNA capable of treating Huntington's disease is selected from siRNA of mHTT gene or a nucleic acid molecule encoding the RNA.

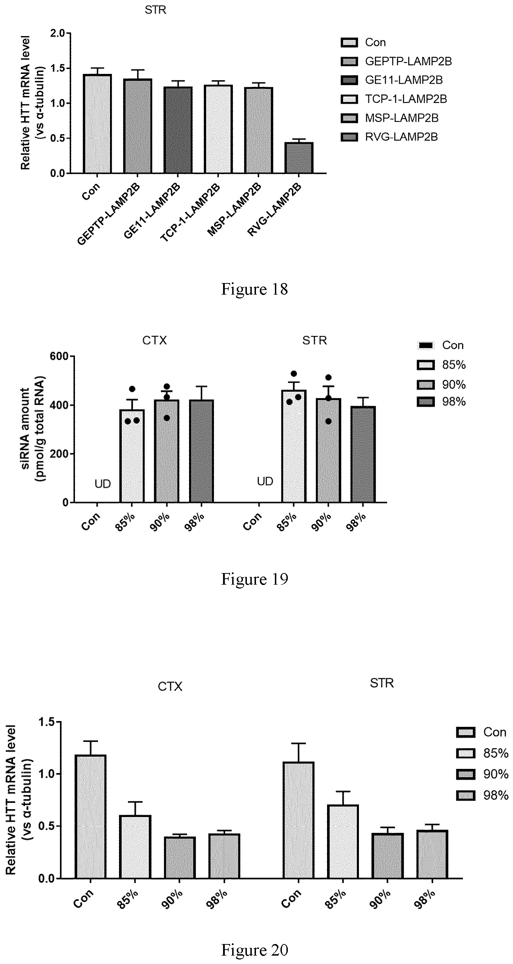

Taking AUUUAGUAGCCAACUAUAGAA as an example, we designed AUUUAGUAGCCAACUAUAUGA (85%), AUUUAGUAGCCAACUAUAACC (90%) and AUUUAGUAGCCAACUAUAGAC (98%), all of which have good gene silencing effect. They are respectively loaded with three homologous sequences of siRNA, and their in vivo enrichment and therapeutic effects on Huntington's disease are shown in Figures 19-20.

The number of effective RNA sequences capable of treat Huntington's disease is one, two or more.

Taking the combined use of "siRNA1" and "siRNA2" on the same virus vector as an example, the functional structural region of the virus vector can be expressed as (promoter-siRNA 1)-linker-(promoter-siRNA 2)-linker-(promoter-targeting tag), or (promoter-targeting tag-siRNA1)-linker-(promoter-targeting tag-siRNA2), or (promoter-siRNA1)-linker-(promoter-targeting tag-siRNA 2), etc.

More specifically, the functional structural region of the virus vector can be expressed as (5'-promoter-5' flanking sequence-siRNA 1-loop sequence-compensation sequence-3' flanking sequence)-linker-(5'-promoter-5'flanking sequence-siRNA 2-loop sequence-compensation sequence-3' flanking sequence)-linker-(5'-promoter-targeting tag), or (5'-promoter-targeting tag-5' flanking sequence-siRNA 1-loop sequence-compensation sequence-3' flanking sequence)-linker-(5'-promoter-targeting tag-5' flanking sequence-siRNA 2-loop sequence-compensating sequence-3' flanking sequence), or (5'-promoter-5' flanking sequence-siRNA 1-loop sequence-compensation sequence-3' flanking sequence)-linker-(5'-promoter-targeting tag-5' flanking sequence-siRNA 2-loop sequence-compensation sequence-3' flanking sequence), or (5'-promoter-targeting tag-5' flanking sequence-siRNA 1- siRNA 2- loop sequence-compensation sequence-3' flanking sequence), etc. Other situations can be deduced in this way and will not be repeated here. The above linker can be "sequence 1-sequence 2-sequence 3" or "sequence 4", and a bracket indicates a complete circuit.

Preferably, the above RNA can also be obtained by ribose modification, preferably 2' fluoropyrimidine modification of RNA sequence (siRNA, shRNA or miRNA) therein. 2' fluoropyrimidine modification is to replace 2'-OH of pyrimidine nucleotide on siRNA, shRNA or miRNA with 2'-F, which can make RNase in human body difficult to recognize siRNA, shRNA or miRNA, thus increasing the stability of RNA during transport in the body.

Specifically, the liver will devour exogenous viruses, and up to 99% of exogenous viruses will enter the liver. Therefore, when viruses are used as vectors, they can be enriched in the liver tissue without specific design. Then, the virus vector is opened and RNA molecules (siRNA, shRNA or miRNA) are released. The liver tissue spontaneously wraps the above RNA molecules into exosomes, and these exosomes become RNA delivery mechanisms.

Preferably, in order to make the RNA delivery mechanism (exosome) have the ability of "precise guidance", we design a targeting tag in the virus vector injected into the body, and the targeting tag will also be assembled into the exosome by the liver tissue. Especially when certain specific targeting tags are selected, the targeting tag can be inserted into the surface of the exosome, thus becoming a targeting structure capable of guiding the exosome, which greatly improves the accuracy of the RNA delivery mechanism described in the invention, and on the one hand, can greatly reduce the amount of virus vector to be introduced, and on the other hand, it can greatly improve the efficiency of potential medicament delivery.

The targeting tag is selected from peptide, protein or antibody with targeting function. The selection of targeting tag is a process that requires creative labor. On the one hand, it is necessary to select the available targeting tag according to the target tissue, and on the other hand, it is necessary to ensure that the targeting tag can stably exist on the surface of exosomes, so as to achieve the targeting function. At present, the targeting tags that have been screened include: targeting peptides, targeting proteins and antibodies. Among them, targeting peptides include, but are not limited to, RVG targeting peptide (nucleotide sequence shown in SEQ ID NO: 1), GE11 targeting peptide (nucleotide sequence shown in SEQ ID NO: 2), PTP targeting peptide (nucleotide sequence shown in SEQ ID NO: 3), TCP-1 targeting peptide (nucleotide sequence shown in SEQ ID NO: 4) and MSP targeting peptide (nucleotide sequence shown in SEQ ID NO: 5). Targeting proteins include, but are not limited to, RVG-LAMP2B fusion protein (nucleotide sequence shown in SEQ ID NO: 6), GE11-LAMP2B fusion protein (nucleotide sequence shown in SEQ ID NO: 7), PTP-LAMP2B fusion protein (nucleotide sequence shown in SEQ ID NO: 8), TCP-1-LAMP2B fusion protein (nucleotide sequence shown in SEQ ID NO: 9) and MSP-LAMP2B fusion protein (nucleotide sequence shown in SEQ ID NO: 10). In this example, the targeting tag is preferably an RVG targeting peptide or an RVG-LAMP2B fusion protein that can accurately target brain tissue.

The in vivo enrichment and the therapeutic effect on Huntington's disease of RVG-LAMP2B fusion protein on adenovirus vector are shown in Figure. 18, and its therapeutic effect is reflected by the level of HTT mRNA.

In addition, in order to achieve the goal of accurate delivery, we have experimented with a variety of virus vector carrying schemes, and obtained another optimized scheme: the virus vector can also be composed of a variety of viruses with different structures, one of which contains promoters and targeting tags, and the other viruses contain promoters and RNA fragments. That is, loading the targeting tag and RNA fragment into different virus vectors, and injecting the two virus vectors into the body, the targeting effect is not worse than that produced by loading the same targeting tag and RNA fragment into one virus vector.

More preferably, when two different virus vectors are injected into the host, the virus vector containing RNA sequence can be injected first, and then the virus vector containing targeting tag can be injected after 1-2 hours, so that better targeting effect can be achieved.

The delivery systems described above can all be used in mammals, including humans.

The RNA delivery system for the treatment of Huntington's disease provided in this example takes the virus as a carrier. As a mature injection material, the virus vector has been fully verified for its safety and reliability, and it exhibits excellent medicament-making property. The RNA sequences that ultimately effect are delivered enclosed within endogenous exosomes, leading to the absence of any immune reactions, eliminating the need to validate the safety of these exosomes. The delivery system can deliver all kinds of small molecular RNA with strong versatility. Moreover, the preparation of virus vectors is much cheaper and more economical than the preparation of exosomes, proteins, polypeptides and other substances. After self-assembly in vivo, the RNA delivery system for the treatment of Huntington's disease provided in this example can tightly binds with AGO₂ and enriched as a complex (exosome), which not only can prevent its premature degradation and maintain its stability in circulation, but also is beneficial to the absorption of recipient cells, cytoplasmic release and lysosomal escape, and only a low dose is required.

### Example 2

On the basis of Example 1, this example provides a medicament. The medicament comprises a viral vector, the viral vector carries RNA fragments capable of treating Huntington's disease, the viral vector is capable of enrichment in organ tissues of a host and endogenously and spontaneously forming a complex containing the RNA fragments capable of treating Huntington's disease in the organ tissues of the host, and the complex can deliver the RNA fragments into a target tissue to treat Huntington's disease.

Optionally, the RNA fragment contains one, two or more specific RNA sequences with medical significance, and the RNA sequences are siRNA, shRNA or miRNA with medical significance.

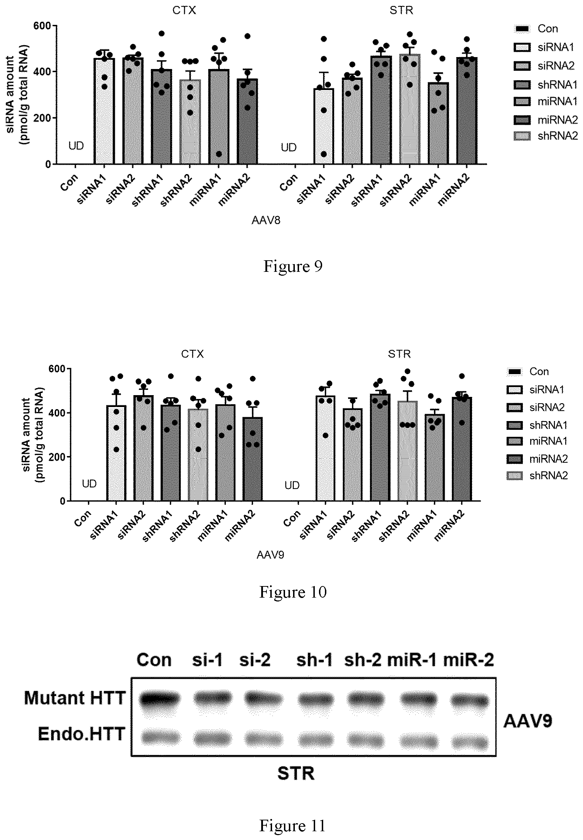

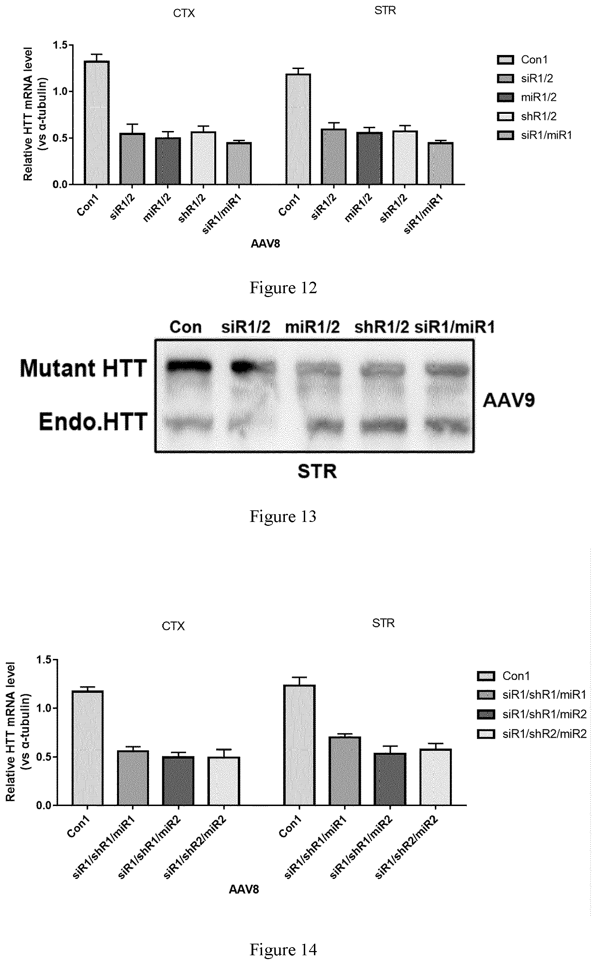

Adenovirus-associated virus types 8 and 9 are used as virus vectors, in the case where they carry one or more RNA fragments, they have in vivo enrichment, self-assembly and treatment effects on Huntington's disease. Figures 9-11 show the data results presented by RNA fragments carrying six separate RNA sequences. Figures 12-13 show the data results presented by the vector carrying four groups of RNA fragments containing any two RNA sequences. Figures 14-15 show the therapeutic data presented by the vector carrying three groups of RNA fragments containing any three RNA sequences.

The sequence is shown in Table 3 below.

| **Name** | **Sequence** |
|---|---|
| siRNA-1 | TTGGTAGCTGAAAGTTCTTGTTTTGGCCACTGACTGACAAGAACTTTCAGCTACCAA |
| siRNA-2 | GAGGAGCCGCTGCACCGACCAGTTTTGGCCACTGACTGACTGGTCGGTAGCGGCTC CTC |
| shRNA-1 | TTGGTAGCTGAAAGTTCTTGTTTTGGCCACTGACTGACAAGAACTTTCAGCTACCAA |
| shRNA-2 | GAGGAGCCGCTGCACCGACCAGTTTTGGCCACTGACTGACTGGTCGGTAGCGGCTC CTC |
| miRNA-1 (miR-137) | ACGGGTATTCTTGGGTGGATAATGTTTTGGCCACTGACTGACTTATTGCTTAAGAATA CGCGTAG |
| miRNA-2 (miR-214) | TGCCTGTCTACACTTGCTGTGCGTTTGGCCACTGACTGACACAGCAGGCACAGACA GGCAGT |

Optionally, the viral vector comprises a promoter and a targeting tag, the targeting tag is capable of forming a targeting structure for the complex in the organ tissues of the host, the targeting structure is located on the surface of the complex, the complex can find and bind to target tissues through the targeting structure, to deliver the RNA fragments into the target tissues.

For the explanation of the above viral vectors, RNA fragments, targeting tags, etc. in this example, please refer to Example 1, and will not be repeated here.

The medicament can enter the human body by oral administration, inhalation, subcutaneous injection, intramuscular injection or intravenous injection, and then be delivered to the target tissue through the RNA delivery system described in Example 1 to exert a therapeutic effect.

The medicament can also be used in combination with other medicaments for the treatment of Huntington's disease to enhance the therapeutic effect, such as Tetrabenazine, Olanzapine, Risperidone, Tiapride, Baclofen, Benzodiazepines, Selective Serotonin Reuptake Inhibitors, Mirtazapine, Amantadine, Sodium Valproate, Botulinum Toxin, and the like.

The medicament in this example may also comprise a pharmaceutically acceptable carrier, which includes but is not limited to diluents, buffers, emulsions, encapsulating agents, excipients, fillers, adhesives, sprays, transdermal absorption agents, wetting agents, disintegrants, absorption accelerators, surfactants, colorants, flavoring agents, adjuvants, desiccants, adsorption carriers, etc.

The dosage form of the medicament provided in this example may be tablets, capsules, powders, granules, pills, suppositories, ointments, solutions, suspensions, lotions, gels, pastes, etc.

The medicament provided in this example takes the virus as a carrier. As a mature injection material, the virus vector has been fully verified for its safety and reliability, and it exhibits excellent medicament-making property. The RNA sequences that ultimately effect are delivered enclosed within endogenous exosomes, leading to the absence of any immune reactions, eliminating the need to validate the safety of these exosomes. The medicament can deliver all kinds of small molecular RNA with strong versatility. Moreover, the preparation of virus vectors is much cheaper and more economical than the preparation of exosomes, proteins, polypeptides and other substances. After self-assembly in vivo, the medicament provided in the present application can tightly binds with AGO₂ and enriched as a complex (exosome), which not only can prevent its premature degradation and maintain its stability in circulation, but also is beneficial to the absorption of recipient cells, cytoplasmic release and lysosomal escape, and only a low dose is required.

### Example 3

On the basis of Example 1 or 2, use of an RNA delivery system in the manufacture of a medicament for treating a disease is for the treatment of Huntington's disease is provided in this example. In this example, the use of RNA delivery system in Huntington's disease treatment is explained in detail with the following experiments.

In this example, AAV-5 type adeno-associated virus with high affinity for the liver was used to encapsulate the HTT siRNA system (AAV-CMV-RVG-siR^{mHTT}/AAV-CMV-RVG-siRm^{HTT}). Mice were injected with 100 µL of AAV solution with a titer of 10¹² V g/ml through the tail vein. The in vivo expression of the AAV system was monitored by small animal in vivo imaging, and after 3 weeks, the AAV system was stably expressed in the body, especially in the liver.

Mice was then selected for modeling. After the completion of modeling, mice were injected with PBS buffer/AAV-CMVscrR/A AV-CMVsiR^{mHTT}/AAV-CMV-RVG-siR^{mHTT} to obtain PBS group/AAV-CMV-scrR group/AAV-CMV-siR^{mHTT} group/AAV-CMV-RVG-siR^{mHTT} group. After the above solution was injected into the tail vein, the plasma exosomes were isolated, labeled with PKH26 dye, and co-cultured with cells to observe the absorption of exosomes by cells. The results are as follows.

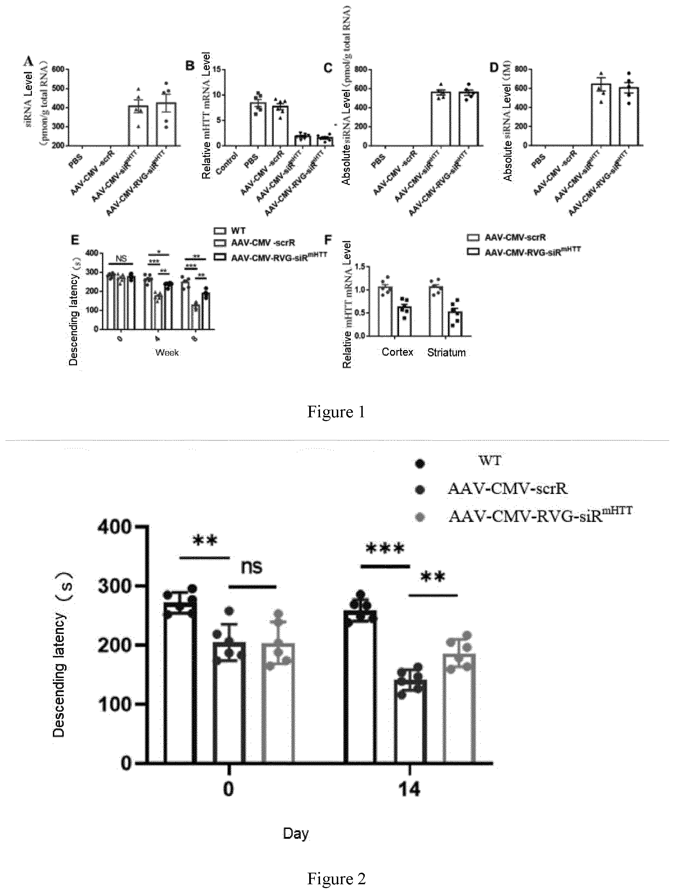

As shown in Figure 1A, the figure shows the comparison of siRNA levels in plasma exosomes of mice in each group. It can be seen that the levels of siRNA in plasma exosomes of mice in the AAV-CMV-siR^{mHTT} group and AAV-CMV-RVG-siR^{mHTT} group were higher.

As shown in Figure 1B, this figure shows the comparison of the relative mHTT mRNA levels of mice in each group after co-culturing the mouse plasma exosomes with cells. It can be seen that the mice in the AAV-CMV-siR^{mHTT} group and AAV-CMV-RVG-siR^{mHTT} group had lower relative mHTT mRNA levels, indicating that AAV-CMV-siR^{mHTT} and AAV-CMV-RVG-siR^{mHTT} could reduce HTT mRNA levels, meaning siRNA assembled into exosomes could still exert gene silencing function.

As shown in Figure 1C, this figure shows the comparison of absolute levels of siRNA in mouse liver. It can be seen that the absolute levels of siRNA of mice in the AAV-CMV-siR^{mHTT} group and AAV-CMVRVG-siR^{mHTT} group were higher.

As shown in Figure 1D, this figure shows the comparison of absolute levels of siRNA in mouse plasma. It can be seen that the absolute levels of siRNA of mice in the AAV-CMV-siR^{mHTT} group and AAV-CMVRVG-siR^{mHTT} group were higher.

As shown in Figure 1E, this figure shows the comparison of the descending latency of wild-type mice (WT) and mice in the AAV-CMV-scrR group and AAV-CMVRVG-siR^{mHTT} group. It can be seen that at 0 weeks, the descending latency of mice in the three group was relatively consistent. At the 4th and 8th weeks, the descending latency of mice in the CMV-scrR group was the shortest.

As shown in Figure 1F, this figure shows the comparison of the relative mHTT mRNA levels in the cortex and striatum of mice in the AAV-CMV-scrR group and AAV-CMV-RVG-siR^{mHTT} group. It can be seen that whether in the cortex or the striatum, the mHTT mRNA levels of mice in the AAV-CMVRVG-siR^{mHTT} group were lower than those in the AAV-CMV-scrR group.

Eight-week-old N17182Q mice were selected for modeling, and AAV-CMVscrR/AAV-CMV-RVG-siR^{mHTT} was injected into the mice after modeling to form AAV-CMV-scrR group/AAV-CMVRVG-siR^{mHTT} group. The rotation test was carried out on Day 0 and Day 14, and the mice were killed for analysis after 14 days.

As shown in Figure 2, which shows the comparison of the descending latency of wild-type mice and the mice in AAV-CMV-scrR group and AAV-CMV-RVG-siR^{mHTT} group. It can be seen that the descending latency of mice in AAV-CMV-scrR group and AAV-CMV-RVG-siR^{mHTT} group is relatively consistent on Day 0, and on Day 14, the descending latency of mice in AAV-CMV-scrR group was significantly shortened.

The above experiments can demonstrate that intravenous injection of AAV-CMV-RVG-siR^{mHTT} can help reduce mHTT protein and toxic aggregates in the striatum and cortex, thereby exerting a therapeutic effect on Huntington's disease.

The terms "upper", "lower", "front", "back", "left", "right", etc. used herein are only used to express the relative positional relationship between related parts, but not to limit the absolute position of these related parts.

The terms "first", "second", etc. used herein are only used to distinguish each other, but do not indicate the degree of importance, order, or the prerequisite for each other's existence.

The terms "equal", "identical", etc. used herein are not limitations in a strict mathematical and/or geometric sense, but also include errors that are understandable by those skilled in the art and allowed in manufacturing or use.

Unless otherwise stated, numerical ranges herein include not only the entire range within both endpoints, but also several subranges subsumed therein.

The preferred specific embodiments and examples of the present application have been described in detail above in conjunction with the accompanying drawings. However, the present application is not limited to the above-mentioned embodiments and examples. Within the scope of knowledge possessed by those skilled in the art, various changes can be made without departing from the concept of the present application.

## Claims

1. An RNA delivery system for the treatment of Huntington's disease, wherein the system comprises a viral vector, the viral vector carries RNA fragments capable of treating Huntington's disease, the viral vector is capable of enrichment in organ tissues of a host and endogenously and spontaneously forming a complex containing the RNA fragments capable of treating Huntington's disease in the organ tissues of the host, and the complex is capable of delivering the RNA fragments into a target tissue to treat Huntington's disease.

2. The RNA delivery system for the treatment of Huntington's disease according to claim 1, wherein the viral vector is an adenovirus-associated virus.

3. The RNA delivery system for the treatment of Huntington's disease according to claim 2, wherein the adenovirus-associated virus is selected from adenovirus-associated virus type 5, adenovirus-associated virus type 8, or adenovirus-associated virus type 9.

4. The RNA delivery system for the treatment of Huntington's disease according to claim 1, wherein the RNA fragments comprise one, two, or more specific RNA sequences with medical significance, and the RNA sequence is selected from siRNA, shRNA, or miRNA with medical significance.

5. The RNA delivery system for the treatment of Huntington's disease according to claim 1, wherein the viral vector comprises a promoter and a targeting tag, the targeting tag is capable of forming a targeting structure for the complex in the organ tissues of the host, the targeting structure is located on the surface of the complex, the complex is capable of finding and binding to target tissues through the targeting structure, to deliver the RNA fragments into the target tissues.

6. The RNA delivery system for the treatment of Huntington's disease according to claim 5, wherein the viral vector comprises any one of the following circuits or the combination of several circuits: promoter-RNA fragment, promoter-targeting tag, promoter-RNA fragment-targeting tag; each viral vector comprises at least one RNA fragment and one targeting tag, and the RNA fragment and targeting tag are located in the same circuit or different circuits.

7. The RNA delivery system for the treatment of Huntington's disease according to claim 6, wherein the viral vector further comprises a flanking sequence, a compensation sequence and a loop sequence that facilitate the folding into a correct structure and the expression of the circuit, the flanking sequences comprises 5' flanking sequences and 3' flanking sequences;
the viral vector comprises any one of the following circuits or the combination of several circuits: 5'-promoter-5' flanking sequence-RNA fragment-loop sequence-compensation sequence-3' flanking sequence, 5'-promoter-targeting tag, 5'-promoter-targeting tag-5' flanking sequence-RNA fragment-loop sequence-compensation sequence-3' flanking sequence.

8. The RNA delivery system for the treatment of Huntington's disease according to claim 7, wherein the 5' flanking sequence has a sequence that is identical to or more than 80% homologous with ggatcctggaggcttgctgaaggctgtatgctgaattc;
the loop sequence has a sequence that is identical to or more than 80% homologous with gttttggccactgactgac;
the 3' flanking sequence has a sequence that is identical to or more than 80% homologous with accggtcaggacacaaggcctgttactagcactcacatggaacaaatggcccagatctggccgcactcgag;
the compensation sequence has a reverse complementary sequence to the RNA fragment in which any 1 to 5 bases have been deleted.

9. The RNA delivery system for the treatment of Huntington's disease according to claim 6, wherein in the case where there are at least two circuits in the virus vector, adjacent circuits are connected by a sequence consisting of sequences 1-3;
wherein sequence 1 is CAGATC, sequence 2 is a sequence consisting of 5-80 bases, and sequence 3 is TGGATC.

10. The RNA delivery system for the treatment of Huntington's disease according to claim 9, wherein in the case where there are at least two circuits in the virus vector, adjacent circuits are connected by a sequence that is identical to or more than 80% homologous with sequence 4;
wherein sequence 4 is
CAGATCTGGCCGCACTCGAGGTAGTGAGTCGACCAGTGGATC.

11. The RNA delivery system for the treatment of Huntington's disease according to claim 1, wherein the organ tissue is liver, and the complex is exosome.

12. The RNA delivery system for the treatment of Huntington's disease according to claim 5, wherein the targeting tag is selected from targeting peptides or targeting proteins with targeting functions.

13. The RNA delivery system for the treatment of Huntington's disease according to claim 12, wherein the targeting peptides include RVG targeting peptide, GE11 targeting peptide, PTP targeting peptide, TCP-1 targeting peptide, MSP targeting peptide;
the targeting proteins include RVG-LAMP2B fusion protein, GE11-LAMP2B fusion protein, PTP-LAMP2B fusion protein, TCP-1-LAMP2B fusion protein, MSP-LAMP2B fusion protein.

14. The RNA delivery system for the treatment of Huntington's disease according to claim 4, wherein the length of the RNA sequence is 15-25 nucleotides.

15. The RNA delivery system for the treatment of Huntington's disease according to claim 14, wherein the RNA capable of treating Huntington's disease is selected from siRNA of mHTT gene, or a sequence that is identical to or more than 80% homologous with the above sequence, or a nucleic acid molecule encoding the above RNA.

16. The RNA delivery system for the treatment of Huntington's disease according to claim 15, wherein the siRNA of the mHTT gene includes UAUGUUUUCACAUAUUGUCAG, AUUUAGUAGCCAACUAUAGAA, AUGUUUUUCAAUAAAUGUGCC, UAUGAAUAGCAUUCUUAUCUG, UAUUUGUUCCUCUUAAUACAA, other sequences that inhibit mHTT gene expression, and sequences that are identical to or more than 80% homologous with the above sequences.

17. The RNA delivery system for the treatment of Huntington's disease according to claim 1, wherein the delivery system is a delivery system for use in a mammal including human.

18. Application of the RNA delivery system for the treatment of Huntington's disease according to any one of claims 1-17 in the manufacture of a medicament for treating a disease.

19. The application according to claim 18, wherein the medicament is a medicament for the treatment of Huntington's disease and related diseases, and the medicament is administered by oral administration, inhalation, subcutaneous injection, intramuscular injection, or intravenous injection.
